Europäisches Patentamt

European Patent Office

Office européen des brevets

(19)

(11) EP 0 834 825 B1

(12) **EUROPÄISCHE PATENTSCHRIFT**

(45) Veröffentlichungstag und Bekanntmachung des Hinweises auf die Patenterteilung:
**14.04.2004 Patentblatt 2004/16**

(51) Int Cl.[7]: **G06F 19/00**, A61B 5/00

(21) Anmeldenummer: **97250234.8**

(22) Anmeldetag: **11.08.1997**

(54) **Verfahren und Anordnung zur individualspezifischen Anpassung eines Simulationsmodells zur prospektiven Bestimmung der Blutzuckerkonzentration, der Insulinwirkung und der Nahrungsresorption**

Method and device for the individual-specific adaption of a simultion model for the determination of the daily profiles of blood sugar concentration, the insulin effect and the food absorption

Méthode et dispositif pour l'adaption spécifique à un individu d'un modèle de simulation destiné à déterminer le profil journalier de la concentration du sucre dans le sang, de l'action de l'insuline et de la résorption des aliments

(84) Benannte Vertragsstaaten:
**DE GB**

(30) Priorität: **27.08.1996 DE 19634577**

(43) Veröffentlichungstag der Anmeldung:
**08.04.1998 Patentblatt 1998/15**

(73) Patentinhaber:
- **Salzsieder, Eckhard, Dr.**
  **17495 Karlsburg (DE)**
- **Rutscher, Alexander, Dipl.-Ing.**
  **17495 Züssow (DE)**

(72) Erfinder:
- **Salzsieder, Eckhard, Dr.**
  **17495 Karlsburg (DE)**
- **Rutscher, Alexander, Dipl.-Ing.**
  **17495 Züssow (DE)**

(74) Vertreter: **Meyerhöfer, Dietmar, Dipl.-Ing. Patentanwalt,**
**Rudolf-Petershagen-Allee 12**
**17489 Greifswald (DE)**

(56) Entgegenhaltungen:
DD-A- 230 730

- LEHMANN E D ET AL: "An integrated approach for the computer-assisted treatment of diabetic patients on insulin" MEDICAL INFORMATICS, APRIL-JUNE 1992, UK, Bd. 17, Nr. 2, ISSN 0307-7640, Seiten 105-123, XP002052427

- DEUTSCH T ET AL: "UTOPIA: a consultation system for visit-by-visit diabetes management" MEDICAL INFORMATICS, OCT.-DEC. 1996, TAYLOR & FRANCIS, UK, Bd. 21, Nr. 4, ISSN 0307-7640, Seiten 345-358, XP002052428
- FISHER M E ET AL: "Optimal insulin infusion resulting from a mathematical model of blood glucose dynamics" IEEE TRANSACTIONS ON BIOMEDICAL ENGINEERING, APRIL 1989, USA, Bd. 36, Nr. 4, ISSN 0018-9294, Seiten 479-486, XP002052429
- ANDREASSEN S: "Model-based biosignal interpretation" METHODS OF INFORMATION IN MEDICINE, MARCH 1994, GERMANY, Bd. 33, Nr. 1, ISSN 0026-1270, Seiten 103-110, XP002052430
- RUTSCHER A ET AL: "KADIS: model-aided education in type I diabetes" COMPUTER METHODS AND PROGRAMS IN BIOMEDICINE, JAN. 1994, NETHERLANDS, Bd. 41, Nr. 3-4, ISSN 0169-2607, Seiten 205-215, XP002052431
- LEHMANN E.D. ET AL: 'Combining rule-based reasoning and mathematical modelling in diabetes care' ARTIFICIAL INTELLIGENCE IN MEDICINE Bd. 6, 1994, Seiten 137 - 160
- SALZSIEDER E. ET AL: 'Computer-aided systems in the management of type I diabetes: the application of a model-based strategy' COMPUTER METHODS AND PROGRAMS IN BIOMEDICINE Bd. 32, 1990 - 1990, ELSEVIER SCIENCE PUBLISHERS BIOMEDICAL DIVISION, Seiten 215 - 224, XP001012745

## Beschreibung

Die Erfindung betrifft ein Verfahren und eine Anordnung zur individualspezifischen Anpassung eines Simulationsmodells zur prospektiven Bestimmung der Tagesprofile der Blutzuckerkonzentration, der Insulinwirkung und der Nahrungsresorption und findet in der rechnergestützten Medizintechnik für die Gesundheitspflege Anwendung.

Es ist bekannt, dass die aktuelle Leistungsfähigkeit und Lebensqualität von an Diabetes mellitus erkrankten Probanden maßgeblich von einer individuell optimalen Stoffwechselführung abhängen. Deshalb werden ständig verbesserte technische Lösungen zur Optimierung der Stoffwechselführung angestrebt. Diese beinhalten in der Regel Blutzuckerselbstkontrollen und führen somit zu einem verstärkten Einfluss des Probanden auf sein eigenes Behandlungsergebnis.

Die Kenntnis über die Wechselwirkungen zwischen subkutan verabfolgtem Insulin, aufgenommener Nahrung und den individuellen Blutzuckertagesprofilen sind hierbei unerläßlich. Eintritt der Wirkung, Zeitpunkte der Wirkungsmaxima und der Wirkungsdauer in Abhängigkeit von den verabfolgten Insulineinheiten bzw. den Broteinheiten der Nahrung sind Beispiele von Faktoren, die die Dynamik und den Verlauf der täglichen Blutzuckerkonzentration entscheidend mitbestimmen.

Bisher konnte nach Howorka, K. : Functional Insulin Treatment, Principles, Teaching Approach and Practice, Springer-Verlag 1989, das Problem der zeit- und situationsgerechten Zuordnung nur durch langwieriges Ausprobieren verschiedener therapeutischer Maßnahmen durch den an Diabetes mellitus erkrankten Probanden, unterstützt durch allgemeingültige Aussagen, z.B. in Form von Faustregeln oder einfachen Algorithmen gelöst werden.

Durch Berger, M. et al.: Computer simulation of plasma insulin and glucose dynamics after subcutaneous insulin injection, Diabetes Care, Vol. 12, No. 10, Nov./Dec. 1989, ist bereits ein rechnergestütztes Modell zur Bestimmung von Insulin- und Glukose-Profilen beschrieben worden.

Allen diesen Verfahren ist gemeinsam, dass ihre Aussagen nicht auf einen gegebenen an Diabetes mellitus erkrankten Probanden zugeschnitten und somit die individualspezifischen Wechselwirkungen von Insulin, Nahrung und Blutzuckertagesprofil nicht ausreichend erfaßt sind. Deshalb ist das Erfordernis einer individuellen Beschreibung des Einflusses von therapeutischen Maßnahmen auf den Blutzuckerkonzentrationsverlauf bisher nicht gegeben.

Die Anwendung verschiedenster mathematischer Modellansätze des Glukose/Insulin-Stoffwechsels in Form von Differentialgleichungssystemen oder mit Mitteln der künstlichen Intelligenz, wie z. B. neuronalen oder kausal probabilistischen Netzwerken, sollten zur Lösung des Problems beitragen, so beschrieben durch Ruggiero, C. et al.: A qualitative model of the dynamics of blood glucose and its hormonal control, in Computer Methods and Programs in Biomedicine, 40 (1993) 117-130, durch Lehmann, E. D. et al.: Combining rule-based reasoning and mathematical moidelling in diabetes care, in Artificial Intelligence in Medicine 6 (1994) 137-160 und andere.

Die Mängel dieser derzeitigen Entwicklungen liegen zum einen in der Komplexität dieser Modelle, die einen hohen Bedarf an technischen Hilfsmitteln wie Computerkapazität nach sich ziehen und zum anderen im Fehlen nachvollziehbarer Verfahren einer individuellen Anpassung dieser Modelle an die jeweilige Stoffwechselsituation eines an Diabetes mellitus erkrankten Probanden anhand von routinegemäß erfassbaren Daten.

Durch das Patent DD-A-230730 und durch Salzsieder, E. et al.: Computer-aided system in the management of type I diabetes: the application of a model-based strategy, Computer Methods and Programs in Biomedicine, Elsevier Science Publishers Biomedical Division, 1990, Band 32, 215-224, ist ein physiologisch basiertes Modell bekannt geworden, welches sich prinzipiell an die individuelle Stoffwechselsituation eines gegebenen Probanden mit Typ 1 Diabetes anpassen läßt. Hierzu ist jedoch bislang eine ausschließlich für den Typ 1 Diabetes gültige und nur unter klinischen Bedingungen durchzuführende Testprozedur erforderlich. Das beschriebene Identifikationsverfahren ist daher weder für die Anwendung in der ambulanten Stoffwechselführung noch für die Anwendung beim Typ 2 Diabetes, der ca. 90 - 95 % aller Diabetes-Patienten betrifft, geeignet.

Die Aufgabe dieser Erfindung besteht deshalb darin, in einem einfach zu handhabenden Verfahren und einer Anordnung zur Durchführung des Verfahrens die individualspezifische Anpassung eines Simulationsmodells zur prospektiven Bestimmung der Tagesprofile der Blutzuckerkonzentration, der Insulinwirkung und der Nahrungsresorption an die individuelle Stoffwechselsituation eines an Diabetes mellitus erkrankten Probanden zu ermöglichen.

Das effindungsgemäße Verfahren zur individualspezifischen Anpassung eines Simulationsmodells zur prospektiven Bestimmung der Tagesprofile der Blutzuckerkonzentration, der Insulinwirkung und der Nahrungsresorption besteht darin, dass unter Anwendung eines bekannten mathematischen Modells des physiologischen Glukose/Insulin-Stoffwechsels mit den Zustandsgrößen

Blutzuckerkonzentrationsverlauf $\qquad x' = u + G_{exg},$

endogene Glukosebilanzgröße $\qquad u' = - (b_{1 +}b_2)u - b_3 y + b_1(b_0 - G_{exg})$

$$\text{Insulinkonzentrationsverlauf} \qquad y' = -k_1 y + I_{exg},$$

aus einer Vielzahl vorhandener Selbstkontrolldaten eines an Diabetes mellitus erkrankten Probanden, bestehend aus Angaben zur täglichen Insulinapplikation, zur Nahrungsaufnahme und zu den zu diskreten Zeitpunkten oder kontinuierlich gemessenen Blutzuckerwerten sowie zu dem in der diabetologischen Praxis bewährten Body Mass Index (BMI) mittels eines Auswertealgorithmus probandenspezifische Datensätze selektiert werden, welche die in einem frei wählbaren Beobachtungszeitraum von dem Probanden typischer Weise im Tagesablauf verabfolgten Insulinapplikationen und Nahrungsaufnahmen, die Gesamtmengen der typischer Weise täglich verabfolgten basalen und nahrungsbezogenen Insuline und den im gewählten Beobachtungszeitraum typischen Tagesverlauf der gemessenen Blutzuckerwerte beschreiben. Auf der Grundlage dieser probandenspezifischen Datensätze werden dann mittels des Identifikationsgleichungssystems

$$b_0 = \frac{ID_{Basal} * b_3}{b_1 * k_1} = \text{endogene Glukoseproduktion,}$$

$$b_1 = \frac{BMI * ID_{Basal} * GD}{ID_{Nahrung}} = \text{Verstärkungsfaktor für insulinunabhängigen Glukoseumsatz,}$$

$$b_3 = \frac{b_2 * k_1 * GD}{ID_{Nahrung}} = \text{Insulin/Glukose-Wirkfaktor,}$$

mit den bekannten Größen
BMI = Body Mass Index = Körpergewicht [kg] / Körpergröße$^2$ [m$^2$],
$ID_{Basal}$ = täglich verabfolgte Menge an Basalinsulin,
$ID_{Nahrung}$ = täglich verabfolgte Menge an nahrungsbezogenem Insulin,
GD = täglich aufgenommene Nahrungsmenge,
$k_1$ = Zeitkonstante des Insulinsubsystems,
$b_1 + b_2$ = const. = Zeitkonstante des Glukosesubsystems,
welches erstmalig eine funktionale Beziehung zwischen den Selbstkontrolldaten eines diabetischen Probanden und den Parametern des mathematischen Modells des physiologischen Glukose/Insulin-Stoffwechsels herstellt, die Modellparameterwerte $b_0$, $b_1$, $b_2$, $b_3$, $k_1$ des mathematischen Modells des physiologischen Glukose/Insulin-Stoffwechsels individualspezifisch berechnet. Mit den so ermittelten individualspezifischen Modellparameterwerten werden unter Verwendung des gleichen mathematischen Modells des physiologischen Glukose/Insulin-Stoffwechsels in einem ersten Iterationsschritt die initialen Tagesprofile der Blutzuckerkonzentration, der Insulinwirkung und der Nahrungsresorption für den gegebenen Probanden berechnet und anschließend das modelgestützt berechnete Tagesprofil der Blutzuckerkonzentration mit dem zuvor ermittelten typischen Tagesverlauf der gemessenen Blutzuckerwerte verglichen. In Abhängigkeit vom Ergebnis des Vergleichs dieser beiden Blutzuckertagesprofile werden dann die Insulinwirkprofile über eine Rückkopplungsschleife solange iterativ angepaßt, bis durch die nachfolgenden Vergleiche festgestellt wird, das entsprechend der vorgegebenen Abbruchbedingung keine weitere Verbesserung der Anpassung um mehr als 1 % zwischen dem typischen Tagesprofil der gemessenen Blutzuckerwerte und zwei aufeinander folgend modellgestützt berechneten Tagesprofilen der Blutzuckerkonzentration erreicht werden kann. Mit der Erfüllung der Abbruchbedingung ist die optimale Anpassung des Simulationsmodells zur prospektiven Bestimmung der Tagesprofile der Blutzuckerkonzentration, der Insulinwirkung und der Nahrungsresorption an die Selbstkontrolldaten eines gegebenen Probanden gefunden.

Nach Abschluss des Anpassungsverfahrens stehen die so ermittelten probandenspezifischen Tagesprofile der Blutzuckerkonzentration, der Insulinwirkung und der Nahrungsresorption zur grafischen Auswertung oder zur Weiterverwendung, z. B. in rechnergestützten Diabetes Management Programmen zur Verfügung.

Die erfindungsgemäße Anordnung zur individualspezifischen Anpassung eines Simulationsmodells zur prospektiven Bestimmung der Tagesprofile der Blutzuckerkonzentration, der Insulinwirkung und der Nahrungsresorption ist dadurch beschrieben, dass zuerst aus der Vielzahl vorliegenden Selbstkontrolldaten eines an Diabetes mellitus erkrankten Probanden über eine Eingangsschnittstelle mit Input-Modul einer Mikrorechneranordnung zugeführt werden. Das Input-Modul stellt die Schnittstelle zwischen den Selbstkontrolldaten des Probanden und der erfindungsgemäßen Mikrorechneranordnung dar. Die Selbstkontrolldaten werden dazu vorzugsweise durch Auslesen aus kommerziell erhältlichen elektronischen Datenmanagementgeräten bereitgestellt. Dem Input-Modul ist ein Datenaufbereitungs-Modul nachgeschaltet, welches mittels eines Auswertealgorithmus aus den Selbstkontrolldaten probandenspezifische Datensätze generiert, welche die in dem gewählten Beobachtungszeitraum in typischer Weise von dem Probanden im Tagesablauf zumeist angewandte Abfolge der Insulinapplikationen , die typischer Weise pro Tag verabfolgte Gesamtinsulinmenge einschließlich der Relation zwischen basal und nahrungsbezogen applizierten Insulinen, die täglich zugeführten Broteinheiten sowie den BMI , die Zeitpunkte und Mengen der typischer Weise pro Tag aufgenommenen Nahrung und den im betrachteten Zeitraum typischen Tagesverlauf der gemessenen Blutzuckerwerte beschreiben. An das Datenaufbereitungs-Modul sind ein Güte-Modul und ein Modellparameterberechnungs-Modul, ein Simulations-Modul und ein Komparator-Modul geschaltet. Mittels des Modellparameterberechnungs-Moduls erfolgt die Bestimmung der individualspezifischen Modellparameterwerte $b_0$, $b_1$, $b_2$, $b_3$, $k_1$ für einen gegebenen Probanden anhand des erfindungsgemäßen Identifikationsgleichungssystems. Das Simulations-Modul dient der modellgestützten Berechnung der individualspezifischen Tagesprofile der Blutzuckerkonzentration, der Insulinwirkung und der Nahrungsresorption unter Verwendung des bekannten mathematischen Modells zur Beschreibung des physiologischen Glukose/Insulin-Stoffwechsels in Form eines Differentialgleichungssystems.

Mit Hilfe des Komparator-Moduls wird das modellgestützt berechnete Tagesprofil der Blutzuckerkonzentration mit dem typischen Tagesverlauf der gemessenen Blutzuckerkonzentrationswerte verglichen. In Abhängigkeit vom Vergleichsergebnis werden die modellgestützt berechneten Tagesprofile der Blutzuckerkonzentration solange über eine Rückkopplungsschleife vom Ausgang des Komparators an den Eingang des Wirkprofilberechnungs-Moduls und weiter vom Ausgang des Wirkprofilberechnungs-Moduls an den Eingang des Simulations-Moduls gelegt, bis im Komparator-Modul durch Berechnung der minimalen Restquadratsummen ermittelt wird, dass entsprechend der vorgegebenen Abbruchbedingung keine weitere Verbesserung der Anpassung um mehr als 1% zwischen dem typischen Tagesverlauf der gemessenen Blutzuckerkonzentrationswerte und den in zwei aufeinanderfolgenden Iterationszyklen modellgestützt berechneten Tagesprofilen der Blutzuckerkonzentration erreicht wird. Hierzu werden mittels des Wirkprofilberechnungs-Moduls die Insulinwirkprofile ausgehend von Standardprofilen iterativ angepasst und in jedem Iterationszyklus an das Simulations-Modul zur erneuten modellgestützten Berechnung des individualspezifischen Tagesprofils der Blutzuckerkonzentration gelegt. Mit Erfüllung der vorgegebenen Abbruchbedingung ist die optimale Anpassung des Simulationsmodells an die Selbstkontrolldaten eines gegebenen Probanden vollzogen. Die modellgestützt berechneten individualspezifischen Tagesprofile der Blutzuckerkonzentration, der Insulinwirkung und der Nahrungsresorption werden abschließend an ein Output-Modul gelegt und stehen hier für die grafische Darstellung oder über die Ausgangsschnittstelle der erfindungsgemäßen Mikrorechneranordnung für die Weiterverarbeitung wie beispielsweise in rechnergestützten Diabetes Management Programmen zur Verfügung.

Erfindungsgemäß ist somit möglich, allein aus routinegemäß erhobenen Selbstkontrolldaten eines an Diabetes mellitus erkrankten Probanden ein Simulationsmodell zur Bestimmung der Tagesprofile der Blutzuckerkonzentration, der Insulinwirkung und der Nahrungsresorption individualspezifisch an die Stoffwechselsituation des gegebenen Probanden anzupassen und damit die für jede Art der Optimierung der Stoffwechselführung bedeutungsvolle Wechselwirkung zwischen den wichtigsten therapeutischen Einflussgrößen Insulin und Nahrung und den resultierenden individuellen Blutzuckerverlauf hinreichend genau zu beschreiben. Dabei finden die bisher bekannten notwendigen klinischen Tests, die sowohl eine große Belastung für den Probanden als auch eine für die Routineanwendung ungeeigneten Aufwand darstellen, keine Anwendung mehr. Das erfindungsgemäße Verfahren ist insbesondere geeignet, in rechnergestützte Diabetes Management Programme integriert zu werden und lässt aufgrund der einfachen Handhabung eine breite Anwendung in der Betreuung der an Diabetes mellitus erkrankten Probanden erwarten.

Bei den rechnergestützten Diabetes Management Programmen handelt es sich um eine neuartige, iterative Behandlungsform, die sowohl den Patienten bei der Aneignung von Kenntnissen und Informationen zur täglichen Stoffwechselführung hilfreich ist, als auch die kompetente Beratung von Ärzten und weiteren an der Betreuung teilhabenden Berufsgruppen unterstützt. Die bisher in solchen Programmen allgemeingültigen Aussagen sind durch Anwendung des erfindungsgemäßen Verfahrens nunmehr auf den individuellen Probanden zugeschnitten, was die Effizienz der Diabetes Management Programme nachhaltig verbessert.

Nachstehend soll die Erfindung an einem Ausführungsbeispiel anhand einer Zeichnung näher erläutert werden. Dabei ist dargestellt in

Fig. 1    die erfindungsgemäße Anordnung zur individualspezifischen Anpassung eines Simulationsmodells zur prospektiven Bestimmung der Tagesprofile der Blutzuckerkonzentration, der Insulinwirkung und der Nahrungsresorption

Fig. 2    die grafische Darstellung des Tagesprofils der Blutzuckerkonzentration, der Insulinwirkung und der Nahrungsresorption.

Die erfindungsgemäße Anordnung zur individualspezifischen Anpassung eines Simulationsmodells zur prospektiven Bestimmung der Tagesprofile der Glukosekonzentration, der Insulinwirkung und der Nahrungsresorption besteht aus einer modular aufgebauten Mikrorechneranordnung. An dessen Eingang werden über das Input-Modul 1 Selbstkontrolldaten eines gegebenen diabetischen Probanden zu seinen Blutzuckerwerten aus Datenmanagementsystemen, aus Selbstkontrolltagebüchern und anderen Aufzeichnungssystemen übertragen.

Diese Daten werden in Form des Input-Datensatzes a an das dem Input-Modul 1 nachgeschalteten Datenaufbereitungs-Moduls 2 übergeben.

Der Input-Datensatz a besteht aus den Daten des BMI und den Daten der zu bestimmten Zeitpunkten diskret oder kontinuierlich gemessener Blutzuckerwerte, der Zeitpunkte, der Dosierungen und der Formulationen der Insulinapplikationen und der Zeitpunkte und der Mengen der Nahrungsaufnahme.

Die Struktur des Input-Datensatzes a ist in Tab. 1 dargestellt.

Tab. 1

| Datum | Zeit | Insulinmenge | Formulation | Nahrung | Blutzuckerwert | BMI |
|---|---|---|---|---|---|---|
| aaa | aaa | aaa | A | aaa | aaa | aa |
| bbb | bbb | bbb | B | bbb | bbb | |
| . | . | . | . | . | . | |
| . | . | . | . | . | . | |
| zzz | zzz | zzz | z | zzz | zzz | |

Vom ersten Ausgang des Datenaufbereitungs-Moduls 2 wird der Input-Datensatz a als Datensatz c an den ersten Eingang des nachgeschaltete Güte-Modul 3 gelegt, welches diesen Datensatz mit den dazugehörigen Blutzuk-kerkontrolldaten zur Beschreibung der Güte dieses Therapieregimes verwendet anhand von Maßzahlen der deskriptiven Statistik, welche in dem Güte-Modul 3 abgelegt sind.

Das Datenaufbereitungs-Modul 2 ermittelt aus dem Input-Datensatz a mittels eines Auswertealgorithmus probandenbezogene Datensätze für die typischer Weise im Tagesablauf angewandten Insulinapptikationen, bestehend aus den Zeitpunkten der Insulininjektion, der verabfolgten Insulinmengen und Insulinformulationen.

Das Datenaufbereitungs-Modul 2 erzeugt den probandenbezogenen Datensatz d, der Mengen des typischer Weise pro Tag verabfolgten basal bezogenen Insulins $ID_{basal}$ und pro Tag verabfolgten nahrungsbezogen Insulins $ID_{Nahrung}$, der insgesamt pro Tag zugeführten Nahrungsmenge GD in Broteinheiten BE und den dazugehörigen einzelnen Blutzuckerkonzentrationswerten beschreibt. Der Datensatz d wird vom dritten Ausgang des Datenaufbereitungs-Moduls 2 an den Eingang des Modellparameterbestimmungs-Moduls 4 und an den ersten Eingang des Simulations-Moduls 5 gelegt.

Der probandenbezogene Datensatz e wird aus den für den gegebenen Probanden im betrachteten Zeitintervall typischen Tagesverlauf gemessenen Blutzuckerwerten durch das Datenaufbereitungs-Moduls 2 ermittelt und von dessen vierten Ausgang an den ersten Eingang des Komparator-Moduls 6 geführt.

In Tab. 2 sind Beispiele für die Datensätze d und e angegeben.

Tab. 2

| Datensatz | Zeit | Menge/Wert | Formulation/Bezeichnung/Güte |
|---|---|---|---|
| Insulinapplikationen | 7.00 Uhr | 10 IE | Normal |
| | 7.00 Uhr | 14 IE | Basal |
| | 13.00 Uhr | 8 IE | Normal |
| | 18.00 Uhr | 13 IE | Normal |
| | 22.30 Uhr | 11 IE | Basal |
| d | pro Tag | 25 IE | $ID_{basal}$ |
| | pro Tag | 31 IE | $ID_{Nahrung}$ |
| | pro Tag | 18 BE | GD |
| | | 25,3 | BMI |
| d | 7.30 Uhr | 5 BE | |
| | 11.00 Uhr | 3 BE | |
| | 13.30 Uhr | 6 BE | |
| | 18.30 Uhr | 4 BE | |

Tab. 2   (fortgesetzt)

| Datensatz | Zeit | Menge/Wert | Formulation/Bezeichnung/Güte |
|---|---|---|---|
| e | 6.00 Uhr | 162 mgldl | ± 74 mg/dl |
| | 9.00 Uhr | 192 mg/dl | ± 55 mg/dl |
| | 12.00 Uhr | 102 mg/dl | ± 15 mg/dl |
| | 15.00 Uhr | 79 mg/dl | ± 35 mg/dl |
| | 18.00 Uhr | 193 mg/dl | ± 88 mg/dl |
| | 21.00 Uhr | 119 mg/dl | ± 41 mg/dl |
| | 24.00 Uhr | 131 mg/dl | ± 13 mg/dl |

Nach Abschluss der Anpassung, welche durch das Abbruch-Modul 8 mittels den Abbruchbedingungen entsprechend minimaler Restquadratsummen zwischen charakteristischen Blutzuckerverlauf, charakteristischen Blutzuckerkonzentrationswerten, Tagesprofilen und simulierten Blutzuckerkonzentrationsverlauf über die Verbindung j gesteuert ist, wird der Datensatzes I erzeugt und vom zweiten Ausgang des Komparator-Moduls 6 an den ersten Eingang des Output-Moduls 9 übergeben.

Auf der Grundlage des Datensatzes d werden im Modellparameterberechnungs-Modul 4 mittels des Identifikationsgleichungssystems

$$b_0 = \frac{D_{Basal} * b_3}{b_1 * k_1} = \text{endogene Glukoseproduktion,}$$

$$b_1 = \frac{BMI * ID_{Basal} * GD}{ID_{Nahrung}} = \text{Verstärkungsfaktor für insulinunabhängigen Glukoseumsatz,}$$

$$b_3 = \frac{b_2 * k_1 * GD}{ID_{Nahrung}} = \text{Insulin/Glukose-Wirkfaktor,}$$

mit den bekannten Größen

BMI = Body Mass Index = Körpergewicht [kg] / Körpergröße$^2$ [m$^2$],

$ID_{Basal}$ = täglich verabfolgte Menge an Basalinsulin,

$ID_{Nahrung}$ = täglich verabfolgte Menge an nahrungsbezogenem Insulin,

GD = täglich aufgenommene Nahrungsmenge,

$k_1$ = Zeitkonstante des Insulinsubsystems,

$b_1 + b_2$ = const. = Zeitkonstante des Glukosesubsystems,

welches die Beziehung zwischen den Selbstkontrolldaten und den Parametern des Glukose/Insulin-Stoffwechselmodells herstellt, die Modellparameterwerte $b_0$, $b_1$, $b_2$, $b_3$, $k_1$ des mathematischen Modells des physiologischen Glukose/Insulin-Stoffwechsels individualspezifisch berechnet und in Form des Datensatzes f an den zweiten Eingang des Simulations-Modul 5 gelegt.

Mit diesen individualspezifischen Modellparametem in Form des Datensatzes f am zweiten Eingang des Simulations-Moduls 5 sowie den Informationen und Daten aus dem probandenspezifischen Datensatz d am ersten Eingang des Simulations-Moduls 5 erfolgt dann im Simulations-Modul 5 auf der Grundlage des bekannten Differentialgleichungssystems zur Beschreibung des physiologischen Glukose/Insulin-Stoffwechsels in einem ersten Iterationsschritt die initiale Berechnung der individualspezifischen Tagesprofile der Blutzuckerkonzentration, der Insulinwirkung und der Nahrungsresorption.

Anschließend wird das so berechnete individualspezifische Tagesprofil der Blutzuckerkonzentration in Form des Datensatzes g vom ersten Ausgang des Simulations-Moduls 5 an den zweiten Eingang des Komparator-Moduls 6 geführt und dort mit dem typischen Tagesverlauf der gemessenen Blutzuckerkonzentration in Form des Datensatzes e verglichen. In Abhängigkeit vom Ergebnis dieses Vergleichs, das mit dem Abbruch-Modul 8 festgestellt wird, wird der Datensatz h für das Tagesprofil der Insulinwirkung über eine Rückkopplungsschleife vom dritten Ausgang des Komparator-Moduls 6 an den zweiten Eingang des Wirkprofilberechnungs-Moduls 7 gelegt und solange iterativ angepasst und in Form des Datensatzes i am Ausgang des Wirkprofilberechnungs-Moduls 7 zur erneuten Berechnung der individualspezifischen Tagesprofile der Blutzuckerkonzentration und der Insulinwirkung an den dritten Eingang des Simulations-Moduls 5 geführt, bis im Komparator-Modul 6 durch Berechnung der minimalen Restquadratsumme der Abweichung zwischen dem typischen Tagesverlauf der gemessenen Blutzuckerkonzentration und den in zwei aufeinanderfolgenden Iterationszyklen modellgestützt berechneten Tagesprofilen der Blutzuckerkonzentration festgestellt wird, dass bei Fortsetzung der Iteration keine weitere Verbesserung der Anpassung um mehr als 1% erreicht wird.

Wird entsprechend der vorgegebenen Abbruchbedingung, die mittels des Signals k vom zweiten Ausgang des Komparator-Moduls 6 zur Bewertung der Güte an den zweiten Eingang des Güte-Moduls 3 geliefert wird, keine weitere Verbesserung der Anpassung zwischen dem typischen Tagesverlauf der gemessenen Blutzuckerwerte und den in zwei aufeinanderfolgenden Iterationszyklen modellgestützt berechneten individualspezifischen Tagesprofilen der Blutzuckerkonzentration erreicht, wird mit Erfüllung der vorgegebenen Abbruchbedingung der Datensatz I, welcher das optimal an die individuellen Bedingungen eines Probanden angepasste Tagesprofil der modellgestützt berechneten Blutzuckerkonzentration sowie das Tagesprofil der Nahrungsresorption enthält, vom ersten Ausgang des Komparator-Moduls 6 an den zweiten Eingang des Output-Moduls 9 übergeben.

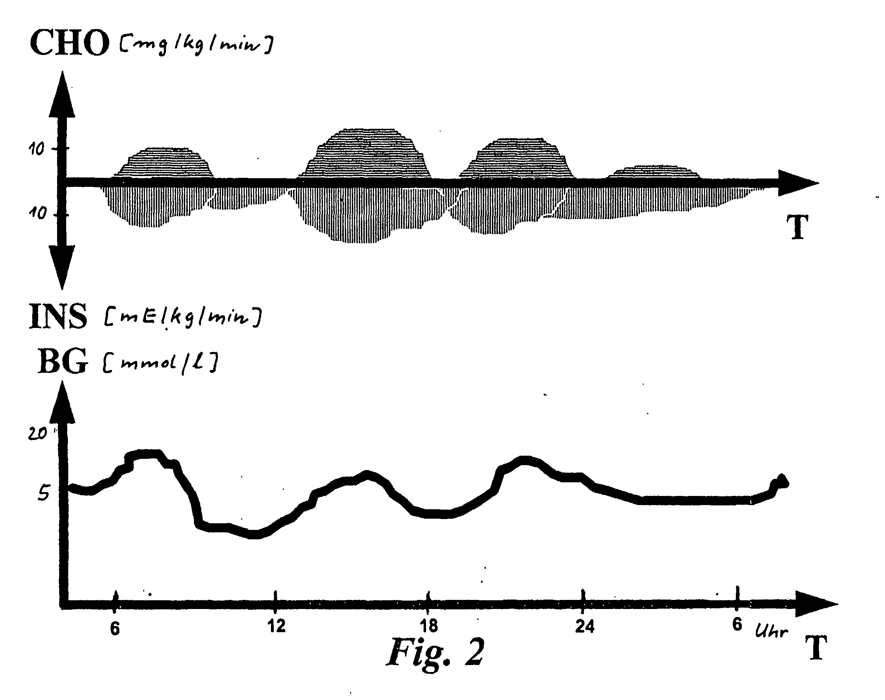

Abschließend werden die probandenspezifischen Parameterwerte des Simulationsmodells und die modellgestützt berechneten individuatspezifischen Tagesprofile der Blutzuckerkonzentration, der Insulinwirkung und der Nahrungsresorption als Datensignal m vom zweiten Ausgang des Simulations-Moduls 5 an den zweiten Eingang des Output-Moduls 9 übergeben und stehen dort für die grafische Darstellung gemäß Fig. 2 zur Verfügung oder können für die Weiterverarbeitung in vorzugsweise rechnergestützten Diabetes Management Programmen über die Output-Schnittstelle der Mikrorechneranordnung abgegriffen werden.

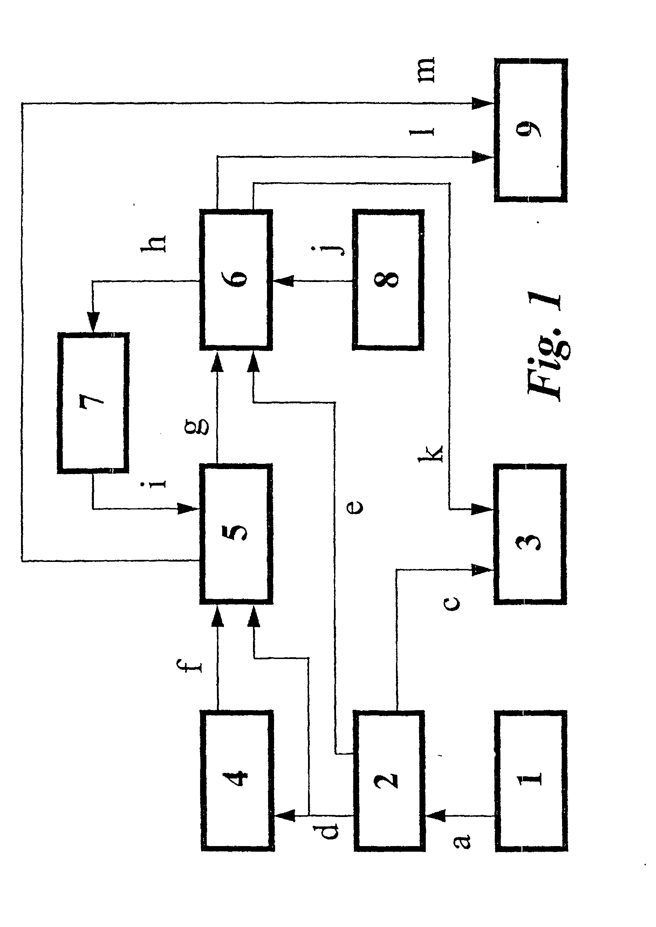

Mit Erfüllung der Abbruchbedingung ist die individualspezifische Anpassung des Simulationsmodells zu Bestimmung der Tagesprofile der Blutzuckerkonzentration, der Insulinwirkung und der Nahrungsresorption an die Selbstkontrolldaten eines gegebenen Probanden gemäß Fig. 1 optimal vollzogen.

In Fig. 2 ist das Diagramm des zeitlichen Verlaufes des individualspezifischen Tagesprofils der Blutzuckerkonzentration BG in mmol/l, der Insulinwirkung INS in mE/kg/min und der Nahrungsresorption CHO in mg/kg/min für einen Probanden im Zeitbereich von 24 Stunden mit Beginn in der Früh dargestellt.


**Patentansprüche**

1. Verfahren zur individualspezifischen Anpassung eines Simulationsmodells für die Bestimmung der Tagesprofile der Blutzuckerkonzentration, der Insulinwirkung und der Nahrungsresorption unter Anwendung eines mathematischen Modells des physiologischen Glukose/Insulin-Stoffwechsels mit den Zustandsgrößen

$$\text{Blutzuckerkonzentrationsverlauf } x' = u + G_{exg},$$

$$\text{endogene Glukosebilanzgröße } u' = -(b_{1} + b_{2})u - b_{3}y + b_{1}(b_{0} - G_{exg})$$

$$\text{Insulinkonzentrationsverlauf } y' = -k_{1}y + I_{exg},$$

**dadurch gekennzeichnet, daß** allein aus den in einem frei wählbaren Beobachtungszeitraum erhobenen Selbstkontrolldaten eines an Diabetes mellitus erkrankten Probanden, bestehend aus Angaben zur täglichen Insulinapplikation und zur Nahrungsaufnahme und aus zu diskreten Zeitpunkten oder kontinuierlich gemessenen Blutzuckerwerten sowie dem BMI, mittels eines Auswertealgorithmus probandenspezifische Datensätze selektiert werden, welche die im gewählten Beobachtungszeitraum typischer Weise im Tagesablauf angewandten Insulinapplikationen, die Gesamtmengen der typischer Weise täglich verabfolgten basalen und nahrungsbezogenen Insuline und aufgenommenen Nahrung sowie den BMI , Zeitpunkt und Menge der typischer Weise im Tagesverlauf vollzogenen Nahrungsaufnahmen und den für den Probanden im gewählten Beobachtungszeitraum typischen Tagesverlauf der gemessenen Blutzuckerkonzentrationswerte darstellen, da sich aus den in den Datensätzen gespeicherten Informationen mittels des Identifikationsgleichungssystems

$$b_{0} = \frac{D_{Basal} * b_{3}}{b_{1} * k_{1}} = \text{endogene Glukoseproduktion,}$$

$$b_1 = \frac{BMI * ID_{Basal} * GD}{ID_{Nahrung}} = \text{Verstärkungsfaktor für insutinunabhängigen Glukoseumsatz,}$$

$$b_3 = \frac{b_2 * k_1 * GD}{ID_{Nahrung}} = \text{Insulin/Glukose-Wirkfaktor,}$$

mit den bekannten Größen
BMI = Body Mass Index = Körpergewicht [kg] / Körpergröße$^2$ [m$^2$],
$ID_{Basal}$ = täglich verabfolgte Menge an Basalinsulin,
$ID_{Nahrung}$ = täglich verabfolgte Menge an nahrungsbezogenem Insulin,
GD = täglich aufgenommene Nahrungsmenge,
$k_1$ = Zeitkonstante des Insulinsubsystems,
$b_1 + b_2$ = const. = Zeitkonstante des Glukosesubsystems,
welches die Beziehung zwischen den Selbstkontrolldaten und den Parametern des Glukose/Insulin-Stoffwechsel-modells herstellt, die Modellparameterwerte $b_0$, $b_1$, $b_2$, $b_3$, $k_1$ des Models des physiologischen Glukose/Insulin-Stoffwechsels individualspezifisch bestimmt werden, daß mit diesen individualspezifischen Modellparameterwerten auf der Grundlage desselben Modells sowie den in den Datensätzen abgespeicherten Informationen über die im Tagesablauf verabfolgten Insuline und aufgenommene Nahrung in einem ersten Iterationsschritt die modellgestützte Berechnung der initialen probandenspezifischen Tagesprofile der Blutzuckerkonzentration, der Insulinwirkung und der Nahrungsresorption erfolgt, daß anschließend das berechnete Blutzuckertagesprofil mit dem typischen Tagesverlauf der gemessenen Blutzuckerwerte verglichen wird, daß in Abhängigkeit von Ausgang dieses Vergleichs die Insulinwirkprofile über eine Rückkopplungsschleife solange iterativ angepaßt werden, bis durch den Vergleich ermittelt wird, daß entsprechend der vorgegebenen Abbruchbedingung keine weitere Verbesserung der Anpassung um mehr als 1% zwischen dem typischen Tagesverlauf der gemessenen Blutzuckerwerte und den in zwei aufeinander folgenden Iterationszyklen modellgestützt berechneten Tagesprofilen der Blutzuckerkonzentration zu erreichen ist, so daß mit Erfüllung der vorgegebenen Abbruchbedingung die optimale Anpassung des Simulationsmodells zur prospektiven Bestimmung der Tagesprofile der Blutzuckerkonzentration, der Insulinwirkung und der Nahrungsresorption an die Selbstkontrolldaten eines gegebenen Probanden vollzogen ist und daß somit die probandenspezifischen Tagesprofile der Blutzuckerkonzentration, der Insulinwirkung und der Nahrungsresorption für die grafische Darstellung und für die vorzugsweise Weiterverwendung in rechnergestützten Diabetes Management Programmen zur Verfügung stehen.

2. Anordnung zur individualspezifischen Anpassung eines Simulationsmodells zur prospektiven Bestimmung der Tagesprofile der Glukosekonzentration, der Insulinwirkung und der Nahrungsresorption, **dadurch gekennzeichnet, dass** in einer modular aufgebauten Mikrorechneranordnung aus den Selbstkontrolldaten eines diabetischen Probanden zu seinen Blutzuckerwerten aus Datenmanagementsystemen, aus Selbstkontrolltagebüchem und anderen Aufzeichnungssystemen, die an den Eingang eines Input-Moduls (1) geführt sind, der Input-Datensatz a, der aus den Daten des BMI und den Daten der zu bestimmten Zeitpunkten diskret oder kontinuierlich gemessener Blutzuckerwerte, der Zeitpunkte, der Dosierungen und der Formulationen der Insulinapplikationen und der Zeitpunkte und der Mengen der Nahrungsaufnahme besteht, erzeugt und an dem nachgeschalteten Datenaufbereitungs-Modul (2) geführt wird dass das Datenaufbereitungs-Modul mittels eines Auswertealgorithmus aus dem Input-Datensatz a probandenspezifische Datensätze generiert, die die typischer Weise im Tagesablauf angewandten Insulinapplikationen, bestehend aus den Zeitpunkten der Insulininjektionen, der verabfolgten Insulinmengen und Insulinformulationen, beschreiben, und die das Datenaufbereitungsmodul an seinem ersten Ausgang als Datensatz c bereitstellt, der an den ersten Eingang eines nachgeschalteten Güte-Moduls (3) mit den dazugehörigen Blutzuckerkontrolldaten zur Beschreibung der Güte dieses Therapieregimes anhand von Maßzahlen der deskriptiven Statistik, welche im Güte-Modul (3) abgelegt sind, gelegt ist, dass das Datenaufbereitungs-Modul den probandenbezogenen Therapieregime-Datensatz d, der Mengen des typischer Weise pro Tag verabfolgten basal bezogenen Insulins $ID_{basal}$ und pro Tag verabfolgten nahrungsbezogen Insulins $ID_{Nahrung}$, der insgesamt pro Tag zugeführten Nahrungsmenge GD in Broteinheiten BE und den dazugehörigen einzelnen Blutzuckerkonzentrationswerten beinhaltet, erzeugt und von seinem dritten Ausgang des Datenaufbereitungs-Moduls an den Eingang eines Modellparameterbestimmungs-Moduls (4) und an den ersten Eingang eines Simulations-Moduls (5) gelegt ist, dass der probandenbezogenen Datensatz e, der durch das Datenaufbereitungs-Modul aus den für den gegebenen Probanden im betrachteten Zeitintervall typischen Tagesverlauf gemessenen Blutzuckerwerten ermittelt ist, von dessen vierten Ausgang an den ersten Eingang eines Komparator-Moduls (6) geführt ist, dass nach Abschluss der Anpassung, welche durch ein Abbruch-Modul (8) mittels den Abbruchbedingungen entsprechend minimaler Restquadratsummen zwischen charakteristischen Blutzuckerverlauf, charakteristischen Blutzuckerkonzentrationswerten, Tagesprofilen und simulierten Blutzuckerkonzentrationsverlauf über das Signal j gesteuert ist, der Datensatzes I erzeugt und vom ersten Ausgang des Komparator-Moduls an den ersten Eingang eines Output-Moduls (9) übergeben wird, dass auf der Grundlage des Datensatzes d im Modellparameterberechnungs-Modul (4) mittels des Identifikationsgleichungssystems

$$b_0 = \frac{D_{Basal} * b_3}{b_1 * k_1} = \text{endogene Glukoseproduktion,}$$

$$b_1 = \frac{BMI * ID_{Basal} * GD}{ID_{Nahrung}} = \text{Verstärkungsfaktor für insulinunabhängigen Glukoseumsatz,}$$

$$b_3 = \frac{b_2 * k_1 * GD}{ID_{Nahrung}} = \text{Insulin/Glukose-Wirkfaktor,}$$

mit den bekannten Größen

BMI = Body Mass Index = Körpergewicht [kg] / Körpergröße$^2$ [m$^2$],

$ID_{Basal}$ = täglich verabfolgte Menge an Basalinsulin,

$ID_{Nahrung}$ = täglich verabfolgte Menge an nahrungsbezogenem Insulin,

GD = täglich aufgenommene Nahrungsmenge,

$k_1$ = Zeitkonstante des Insulinsubsystems,

$b_1 + b_2$ = const. = Zeitkonstante des Glukosesubsystems,

welches die Beziehung zwischen den Selbstkontrolldaten und den Parametern des Glukose/Insulin-Stoffwechselmodells herstellt, die Modellparameterwerte $b_0$, $b_1$, $b_2$, $b_3$, $k_1$ des mathematischen Modells des physiologischen Glukose/Insulin-Stoffwechsels individualspezifisch berechnet werden und als Datensatzes f an den zweiten Eingang des Simulations-Moduls (5) gelegt ist, dass mit diesen individualspezifischen Modellparametern in Form des Datensatzes f sowie den Informationen und Daten aus dem probandenspezifischen Datensatz d im Simulations-Modul (5) auf der Grundlage des Differentialgleichungssystems zur Beschreibung des physiologischen Glukose/Insulin-Stoffwechsels in einem ersten Iterationsschritt die initiale Berechnung der individualspezifischen Tagesprofile der Blutzuckerkonzentration, der Insulinwirkung und der Nahrungsresorption erfolgt und anschließend das so berechnete individualspezifische Tagesprofil der Blutzuckerkonzentration in Form des Datensatzes g vom ersten Ausgang des Simulations-Moduls an den zweiten Eingang des Komparator-Moduls geführt ist und mit dem typischen Tagesverlauf der gemessenen Blutzuckerkonzentration in Form des Datensatzes e verglichen wird, dass in Abhängigkeit vom Ergebnis dieses Vergleichs, das mit dem Abbruch-Modul festgestellt wird, der Datensatz h für das Tagesprofil der Insulinwirkung über eine Rückkopplungsschleife vom Ausgang des Komparator-Moduls an den Eingang des Wirkprofilberechnungs-Moduls (7) gelegt ist und solange iterativ angepasst wird und als Datensatz i am Ausgang des Wirkprofilberechnungs-Moduls (7) zur erneuten Berechnung der individualspezifischen Tagesprofile der Blutzuckerkonzentration und der Insulinwirkung an das dritten Eingang des Simulations-Moduls (5) geführt ist, bis im Komparator-Modul durch Berechnung der minimalen Restquadratsumme der Abweichung zwischen dem typischen Tagesverlauf der gemessenen Blutzuckerkonzentration und den in zwei aufeinanderfolgenden Iterationszyklen modellgestützt berechneten Tagesprofilen der Blutzuckerkonzentration festgestellt wird, dass bei Fortsetzung der Iteration keine weitere Verbesserung der Anpassung um mehr als 1% erreicht wird, dass entsprechend der vorgegebenen Abbruchbedingung, die mittels des Signals k vom zweiten Ausgang des Komparator-Moduls zur Bewertung der Güte an den zweiten Eingang des Güte-Moduls geliefert wird, keine weitere Verbesserung der Anpassung zwischen dem typischen Tagesverlauf der gemessenen Blutzuckerwerte und den in zwei aufeinanderfolgenden Iterationszyklen modellgestützt berechneten individualspezifischen Tagesprofilen der Blutzuckerkonzentration erreicht wird, wird mit Erfüllung der vorgegebenen Abbruchbedingung der Datensatz l , welcher das optimal an die individuellen Bedingungen eines Probanden angepasste Tagesprofil der modellgestützt berechneten Blutzuckerkonzentration sowie das Tagesprofil der Nahrungsresorption enthält, vom zweiten Ausgang des Simulations-Moduls an den ersten Eingang des Output-Moduls übergeben und dass die probandenspezifischen Parameterwerte des Simulationsmodells und die modellgestützt berechneten individualspezifischen Tagesprofile der Blutzuckerkonzentration, der Insulinwirkung und der Nahrungsresorption als Datensignal m an den zweiten Eingang des Output-Moduls übergeben werden, das grafisch dargestellt oder zur Weiterverarbeitung in rechnergestützten Diabetes Management Programmen über die Output-Schnittstelle der Mikrorechneranordnung zur Verfügung stehen.

**Claims**

1. Method for the individual-specific adaptation of a simulation model for the prospective determination of the daily profiles of the blood sugar concentration, the insulin effect and the food absorption with the use of a mathematical model of the physiological glucose/ insulin metabolism with the parameters

$$\text{blood sugar concentration} \qquad x' = u + G_{exg},$$

$$\text{endogenous glucose balance} \qquad u' = - (b_1 + b_2)u - b_3 y + b_1(b_0 - G_{exg})$$

$$\text{variation in insulin concentration} \qquad y' = - k_1 y + I_{exg}$$

**characterized in that** from the self-monitoring data from a test subject suffering from diabetes mellitus collected in a freely selectable observation period, consisting of data concerning the daily insulin administration and concerning the food intake and of the blood sugar values and the BMI measured at discrete time-points or continuously, by means of an evaluation algorithm test subject-specific data records only are selected which represent the insulin administrations typically used in the course of a day in the selected observation period, the total quantities of the basal and diet-related insulin typically administered daily and the food taken and the BMI, time and quantity of the food intakes typically occurring in the course of the day and the daily variation in the measured blood sugar concentration values typical for the test subject in the selected observation period, since the model parameter values $b_0$, $b_1$, $b_2$, $b_3$ and $k_1$ of the model of the physiological glucose/insulin metabolism are determined individual-specifically from the information stored in the data records by means of the identification equation system

$$b_0 = \frac{D_{basal} * b_3}{b_1 * k_1} = \text{endogenous glucose production,}$$

$$b_1 = \frac{BMI * ID_{basal} * GD}{ID_{food}} = \text{multiplication factor for insulin-independent glucose turnover}$$

$$b_3 = \frac{b_2 * k_1 * GD}{ID_{food}} = \text{insulin/glucose action factor}$$

with the known quantities
BMI = body mass index = body weight [kg]/height$^2$ [m$^2$]
$ID_{basal}$ = quantity of basal insulin administered daily
$ID_{food}$ = quantity of food-related insulin administered daily
GD = quantity of food taken daily
$k_1$ = time constant of the insulin subsystem
$b_1 + b_2$ = const. = time constant of the glucose subsystem
which establishes the relationship between the self-monitoring data and the parameters of the glucose/ insulin metabolism model, that with these individual-specific model parameter values on the basis of the same model and the information stored in the data records concerning the insulin administered and food taken in the course of the day in a first iteration step the model-based calculation of the initial test subject-specific blood sugar concentration daily profile, the insulin effect and the food absorption is carried out, that next the calculated blood sugar daily profile is compared with the typical variation in the measured blood sugar values, that depending on the outcome of this comparison the insulin effect profiles are iteratively adapted by means of a feedback loop until it is determined by the comparison that in accordance with the predefined termination condition no further improvement in the adaptation by more than 1% between the typical daily variation in the measured blood sugar values and the daily blood sugar concentration profiles calculated in two consecutive iteration cycles on the basis of the model can be achieved, so that with the fulfilment of the predefined termination condition the optimal adaptation of the simulation model for the prospective determination of the blood sugar concentration daily profile, the insulin effect and the food intake to the self-monitoring data of a given test subject is attained and that thereby the test subject-specific daily profiles of the blood sugar concentration, the insulin effect and the food absorption are available for graphic representation and for the preferably further use in computer-based diabetes management programmes.

2. Device for the individual-specific adaptation of the simulation model for the prospective determination of the daily profiles of blood sugar concentration, the insulin effect and the food absorption, **characterized in that** in a microcomputer array of modular construction from the self-monitoring data from a diabetic test subject concerning his blood sugar values, from data management systems, from self-monitoring daybooks and other record systems, which are passed to the inlet of an input module (1), the input data record a, which consists of the BMI data and the data on the blood sugar values measured discretely at defined time-points or continuously, the times, the dosages and the formulations of the insulin administrations and the times and the amounts of the food intake, is created and passed to the downstream data processing module (2), that from the input data record a by means of an evaluation algorithm the data processing module generates test subject-specific data records, describes, consisting of the times of the insulin injections, the quantities of insulin and administered insulin formulations, and which the data processing module at its first outlet provides as data record c, which is fed into the first inlet of a downstream quality module (3) with the corresponding blood sugar monitoring data for the description of the quality of this therapy regime on the basis of descriptive statistics dimensioning figures, which are filed in the quality module, that the data processing module creates the test subject-based therapy regime data record d, which contains quantities of the basally based insulin $ID_{basal}$ typically administered per day, the food-related insulin $ID_{food}$ administered per day, the total quantity of food supplied per day GD in bread units BU and the corresponding individual blood concentration values and from its third outlet of the data-processing module is connected to the inlet of a model parameter determination module (4) and to the first inlet of a simulation module (5), that the test subject-based data record e, which is determined by the data processing module from the typical daily variation in blood sugar values measured for the given test subject in the time interval under consideration, is passed from its fourth outlet to the first inlet of a comparator module (6), that after termination of the adaptation, which is controlled by a termination module (8) by means of the termination conditions corresponding to least residual sums of squares between characteristic variation in blood sugar, characteristic blood sugar concentration values, daily profiles and simulated variation in blood sugar concentration via the signal j, the data record I is created and transferred from the first outlet of the comparator module to the first inlet of an output module (9), that the model parameter values $b_0$, $b_1$, $b_2$, $b_3$ and $k_1$ of the mathematical model of the physiological glucose/insulin metabolism are determined individual-specifically in the model parameter calculation module on the basis of the data record d by means of the identification equation system

$$b_0 = \frac{D_{basal} * b_3}{b_1 * k_1} = \text{endogenous glucose production,}$$

$$b_1 = \frac{BMI * ID_{basal} * GD}{ID_{food}} = \text{multiplication factor for insulin-independent glucose turnover}$$

$$b_3 = \frac{b_2 * k_1 * GD}{ID_{food}} = \text{insulin/glucose action factor}$$

with the known quantities
BMI = body mass index = body weight [kg]/height$^2$ [m$^2$]
$ID_{basal}$ = quantity of basal insulin administered daily
$ID_{food}$ = quantity of food-related insulin administered daily
GD = quantity of food taken daily
$k_1$ = time constant of the insulin subsystem
$b_1 + b_2$ = const. = time constant of the glucose subsystem
which establishes the relationship between the self-monitoring data and the parameters of the glucose/ insulin metabolism model, and is fed into the second inlet of the simulation model as data record f, that with these individual-specific model parameters in the form of the data record f and the information and data from the test subject-specific data record d in the simulation module on the basis of the differential equation system for the description of the physiological glucose/insulin metabolism in a first iteration step the initial calculation of the individual-specific daily profile of the blood sugar concentration, the insulin effect and the food absorption is carried out, and next the individual-specific blood sugar concentration daily profile thus calculated is passed in the form of the data record g from the first outlet of the simulation module to the second inlet of the comparator module and compared with the typical daily variation in the measured blood sugar concentration in the form of the data record e, that depending on the outcome of this comparison, which is ascertained with the termination module, the data record h for the daily profile of the insulin effect is passed from the outlet of the comparator module to the inlet of the effect profile calculation module (7) via a feedback loop and adapted iteratively and as data record i at the outlet of the effect profile calculation module is passed to the third inlet of the simulation module for the renewed calculation of the individual-specific blood sugar concentration daily profile, until it is determined in the comparison module by calculation of the least residual sum of squares of the deviation between the typical daily profile of the measured blood sugar concentration and the blood sugar concentration daily profiles calculated in two consecutive iteration cycles on the basis of the model, that on continuation of the iteration no further improvement in the adaptation by more than 1% between the typical daily course of the measured blood sugar values is achieved, that in accordance with the predefined termination condition, which is supplied by means of the signal k from the second outlet of the comparator module to the second inlet of the quality module for the quality evaluation, no further improvement of the adaptation between the typical daily variation in the measured blood sugar values and the daily blood sugar concentration profiles calculated in two consecutive iteration cycles on the basis of the model is achieved, with fulfilment of the predefined termination condition the data record l, which contains the blood sugar concentration daily profile calculated on the basis of the model optimally adapted to the individual conditions of a test subject and the daily food absorption profile, is passed from the second outlet of the simulation module to the first inlet of the output module and that the test subject-specific parameter values of the simulation model and the individual-specific daily profiles of the blood sugar concentration, the insulin effect and the food absorption calculated on the basis of the model are transferred as data signal m to the second inlet of the output module, which are available presented graphically or for further processing in computer-based diabetes management programmes via the output interface of the microcomputer array.

**Revendications**

1. Méthode pour l'adaptation spécifique à un individu d'un modèle de simulation destiné pour à déterminer prospective le profil journalier de la concentration du sucre dans le sang, de l'action de l'insuline et de la résorption des aliments par recours à un modèle mathématique du métabolisme physiologique du glucose et de l'insuline qui présente des grandeurs d'état évolution de la

concentration du sucre dans le sang $\quad x' = u + G_{exg}$

bilan du sucre endogène $\quad u' = -(b_1 + b_2)u - b_3 y + b_1(b_0 - G_{exg})$

évolution de la concentration en insuline $\quad y' = -k_1 y + I_{exg}$

**caractérisé en ce qu'**exclusivement à partir des données d'autocontrôle, recueillies pendant une durée d'observation arbitrairement sélectionnée, d'un sujet qui souffre de diabète sucré, constituées d'indications d'administration quotidienne d'insuline et de prise quotidienne d'aliments, ainsi qu'à partir de valeurs du sucre dans le sang mesurées à des instants discrets ou en continu et du BMI, on sélectionne au moyen d'un algorithme d'évaluation des jeux de données spécifiques au sujet, qui représentent les administrations typiques d'insuline au cours de la journée pendant la durée d'observation sélectionnée, les quantités totales d'insuline basale et d'insuline associée aux aliments typiquement administrées chaque jour et d'aliments pris ainsi que le BMI, l'instant et la quantité des prises d'aliments typiques au cours de la journée et l'évolution quotidienne typique des valeurs de la concentration du sucre dans le sang mesurées pendant la durée d'observation, **en ce qu'**à partir des informations conservées dans les jeux de données et au moyen du système d'équations d'identification

$$b_0 = \frac{D_{basal} * b_3}{b_1 * k_1} = \text{production endogène de sucre}$$

$$b_1 = \frac{BMI * ID_{basal} * GD}{ID_{alim}} = \text{coefficient de renforcement de la conversion du sucre par l'intermédiaire de l'insuline}$$

$$b_3 = \frac{b_2 * k_1 * GD}{ID_{alim}} = \text{coefficient d'action de l'insuline sur le glucose}$$

avec les grandeurs connues

$BMI =$ Body Mass Index = poids corporel [kg] / taille du corps$^2$ [m$^2$],

$ID_{basal} =$ quantité d'insuline basale administrée quotidiennement,

$ID_{alim} =$ quantité d'insuline administrée quotidiennement dans les aliments,

$GD =$ quantité journaliére d'aliments pris,

$k_1 =$ constante de temps du sous-système insuline,

$b_1 + b_2 =$ constante = constante de temps su sous-système glucose,

qui établit les relations entre les données d'autocontrôle et les paramètres du modèle du métabolisme du glucose et de l'insuline, on définit les valeurs des paramètres $b_0$, $b_1$, $b_2$, $b_3$, $k_1$ du modèle physiologique du métabolisme du glucose et de l'insuline spécifique à l'individu, **en ce que** dans une première itération, on effectue selon le modèle le calcul des profils journaliers initiaux, spécifiques au sujet, de la concentration du sucre dans le sang, de l'effet de l'insuline et de la résorption des aliments avec ces valeurs spécifiques à l'individu des paramètres du modèle et les informations conservées dans les jeux de données sur l'insuline administrée chaque jour et sur les prises quotidiennes d'aliments, **en ce qu'**ensuite, on compare le profil journalier du sucre dans le sang que l'on a calculé à l'évolution quotidienne des valeurs mesurées du sucre dans le sang, **en ce que** dans une boucle de rétroaction, on adapte de manière itérative les profils d'action de l'insuline en fonction de la sortie de cette comparaison jusqu'à ce que la comparaison détermine que pour la condition d'arrêt prédéterminée, on ne peut plus obtenir d'amélioration supplémentaire de 1 % de l'adaptation des profils quotidiens calculés dans deux itérations successives du modèle à l'évolution quotidienne typique mesurée des valeurs du sucre dans le sang, de sorte que conformément à la condition d'arrêt prédéterminée, le modèle de simulation pour la détermination prospective des profils quotidiens de la concentration du sucre dans le sang, de l'action de l'insuline et de la résorption des aliments est adapté de manière optimale aux données d'autocontrôle d'un sujet donné et qu'ainsi on dispose des profils quotidiens, spécifiques au sujet, de la concentration du sucre dans le sang, de l'action de l'insuline et de la résorption des aliments pour les représenter graphiquement et de préférence les utiliser dans des programmes informatisés de gestion du diabète.

2. Dispositif pour l'adaptation spécifique à un individu d'un modèle de simulation destiné pour à déterminer prospective le profil journalier de la concentration du sucre dans le sang, de l'action de l'insuline et de la résorption des aliments, **caractérisé en ce que** dans un agencement à microordinateur à structure modulaire et à partir des données d'autocontrôle d'un sujet diabétique sur les valeurs du sucre dans son sang, de systèmes de gestion de données, de livres journaliers d'autocontrôle et d'autres systèmes de saisie qui sont amenées à l'entrée d'un module d'entrée (1), on crée le jeu de données d'entrée a qui est constitué des données du BMI, des données des valeurs du sucre dans le sang mesurées à des instants discrets définis ou en continu, des instants des quantités des prises d'aliments et on les transmet au module (2) de préparation des données raccordé en aval, **en ce qu'**au moyen d'un algorithme d'évaluation et à partir du jeu de données d'entrée a, le module de préparation des données génère des jeux de données spécifique au sujet qui sont constitués des instants des injections d'insuline, des quantités et des formulations de l'insuline administrée et qui les décrivent, le module de préparation des données appliquant ces jeux de données sur sa première sortie en tant que jeu de données c qui est appliqué avec les données associées de contrôle du sucre dans le sang à la première entrée d'un module d'évaluation de qualité (3) qui est raccordé en aval et qui décrit la qualité de ce régime thérapeutique à l'aide de nombres descriptifs de mesure statistique qui sont conservés dans le module d'évaluation de qualité, **en ce que** le module de préparation des données crée le jeu de données d du régime thérapeutique spécifique au sujet, qui contient les quantités de l'insuline basale $ID_{basal}$ typiquement administrée chaque jour et de l'insuline $ID_{alim}$ administrée chaque jour dans les aliments, la quantité d'aliments GD globalement apportés chaque jour en unités de pain BE et les valeurs individuelles de la concentration du sucre dans le sang qui y sont associées, ce jeu de données étant appliqué par la troisième sortie du module de préparation des données à l'entrée d'un module (4) de détermination des paramètres du modèle et à la première entrée d'un module de simulation (5), **en ce que** le jeu de données e spécifique au sujet, qui a été défini par le module de préparation des données à partir des valeurs du sucre dans le sang qui ont été mesurées sur le sujet donné pendant l'intervalle de temps concerné de l'évolution journalière typique, est transmis par la quatrième sortie du module de préparation des données à la première entrée d'un module de comparaison (6), **en ce qu'**à la fin de l'adaptation, qui est contrôlée par le signal j d'un module d'arrêt (8) au moyen de la somme des moindres carrés, en correspondance à des conditions d'arrêt, de l'évolution caractéristique du sucre dans le sang, de valeurs caractéristiques de la concentration du sucre dans le sang, de profils journaliers et de l'évolution simulée de la concentration du sucre dans le sang, le jeu de données I est créé et est transmis par la première sortie du module de comparaison à la première entrée d'un module de sortie (9), **en ce que** sur base du jeu de données d et au moyen du système d'équations d'identification

$$b_0 = \frac{D_{basal} * b_3}{b_1 * k_1} = \text{production endogène de sucre}$$

$$b_1 = \frac{BMI * ID_{basal} * GD}{ID_{alim}} = \text{coefficient de renforcement de la conversion du sucre par l'intermédiaire de l'insuline}$$

$$b_3 = \frac{b_2 * k_1 * GD}{ID_{alim}} = \text{coefficient d'action de l'insuline sur le glucose}$$

avec les grandeurs connues
BMI = Body Mass Index = poids corporel [kg] / taille du corps$^2$ [m$^2$],
$ID_{basal}$ = quantité d'insuline basale administrée quotidiennement,
$ID_{alim}$ = quantité d'insuline administrée quotidiennement dans les aliments,
GD = quantité journalière d'aliments pris
$k_1$ = constante de temps du sous-système insuline,
$b_1 + b_2$ = constante = constante de temps su sous-système glucose,
qui établit les relations entre les données d'autocontrôle et les paramètres du modèle du métabolisme du glucose et de l'insuline, les valeurs des paramètres $b_0$, $b_1$, $b_2$, $b_3$, $k_1$ du modèle physiologique du métabolisme du glucose et de l'insuline spécifique à l'individu sont calculées dans le module de calcul des paramètres du modèle et sont appliquées en tant que jeu de données f à la deuxième entrée du module de simulation, **en ce que** dans le module de simulation, avec ces paramètres du modèle spécifiques à l'individu qui se présentent sous la forme du jeu de données f et avec les informations et données qui proviennent du jeu de données d spécifique au sujet, sur base du système d'équations différentielles qui décrit le métabolisme physiologique du glucose et de l'insuline, une première itération effectue le calcul initial des profils journaliers, spécifiques à l'individu, de la concentration du sucre dans le sang, de l'action de l'insuline et de la résorption des aliments, le profil journalier, spécifique à l'individu, ainsi calculé de la concentration du sucre dans le sang est transféré sous la forme du jeu de données g par la première sortie du module de simulation à la deuxième entrée du module de comparaison et est comparé à l'évolution journalière typique de la concentration mesurée du sucre dans le sang qui se présente sous la forme du jeu de données e, **en ce que** le module d'arrêt constate que le jeu de données h du profil journalier de l'action de l'insuline est appliqué dans une boucle de rétroaction par la sortie du module de comparaison à l'entrée du module (7) de calcul des profils d'action, est adapté par itération et transféré en tant que jeu de données i par la sortie du module de calcul des profils d'action à la troisième entrée du module de simulation pour recommencer la calcul des profils journaliers, spécifiques à l'individu, de la concentration du sucre dans le sang et de l'action de l'insuline, jusqu'à ce que par calcul de la somme des moindres carrés de la différence entre l'évolution journalière typique de la concentration du sucre dans le sang et les profils journaliers de la concentration du sucre dans le sang qui sont calculés à l'aide du modèle dans deux cycles itératifs successifs, on constate dans de module de comparaison que l'on ne peut améliorer l'adaptation de plus de 1 % en poursuivant les itérations, **en ce que** conformément à la condition prédéterminée d'arrêt qui est délivrée au moyen du signal k par la deuxième sortie du module de comparaison à la deuxième entrée du module d'évaluation de qualité pour l'évaluation de la qualité, lorsque l'on ne peut obtenir aucune amélioration supplémentaire de l'adaptation des profils journaliers, spécifiques à l'individu, de la concentration du sucre dans le sang qui sont calculés à l'aide du modèle dans deux cycles d'itération successifs, à l'évolution journalière typique des valeurs du sucre dans le sang qui ont été mesurées, lorsque la condition d'arrêt prédéterminée est satisfaite, le jeu de données I qui contient le profil journalier de la concentration du sucre dans le sang, adapté de manière optimale aux conditions individuelles d'un sujet, ainsi que le profil journalier de la résorption des aliments, est transféré par la deuxième sortie du module de simulation à la première entrée du module de sortie, **en ce que** les valeurs, spécifiques au sujet, des paramètres du modèle de simulation et les profils journaliers, spécifiques à l'individu et calculés à l'aide du modèle, la concentration du sucre dans le sang, l'action de l'insuline et la résorption des aliments sont transférés à la deuxième entrée du module de sortie en tant que signal de données m qui est présenté graphiquement ou est délivré par l'interface de sortie de l'agencement de microordinateur pour être utilisé dans des programmes informatisés de gestion du diabète.

*Fig. 1*

EP 0 834 825 B1

CHO [mg/kg/min]

INS [mE/kg/min]

BG [mmol/L]

Fig. 2